# EUROPEAN PATENT SPECIFICATION

(11) **EP 0 869 138 B1**
(45) Date of publication and mention of the grant of the patent: **07.08.2002**
(21) Application number: 96942994.3
(22) Date of filing: 23.12.1996
(51) Int. Cl.: C08F 220/10, C08J 7/04, G02B 1/04

(54) **NOVEL COPOLYMERS FORMED FROM THREE COMPONENTS AND INTRAOCULAR LENSES MADE THEREOF**
NEUE COPOLYMERE AUS DREI KOMPONENTEN UND DARAUS HERGESTELLTE INTRAOKULARE LINSEN
NOUVEAUX COPOLYMERES CONSTITUES DE TROIS COMPOSANTS ET LENTILLES INTRAOCULAIRES FABRIQUEES A PARTIR DE CES COPOLYMERS

(30) Priority: 22.12.1995 CN 95120427
(43) Date of publication of application: 07.10.1998
(73) Proprietor: SANTEN PHARMACEUTICAL CO., LTD., Higashiyodogawa-ku, Osaka-shi, Osaka 533-8651 (JP); Li, Fumian, Beijing 100871 (CN)
(72) Inventor: LI, Fumian, Beijing 100871 (CN)
(74) Representative: Paul, Dieter-Alfred, Dipl.-Ing.
(86) International application number: CN9600115
(87) International publication number: WO9723523

(56) References cited:
- EP-A- 0 234 749
- GB-A- 2 223 230
- JP-A- 5 080 279
- US-A- 5 147 394
- US-A- 5 290 548

## Description

### FIELD OF THE INVENTION

The invention relates to copolymers useful for ocular lens, especially preferable for foldable intraocular lens (foldable IOL).

### BACKGROUND OF THE INVENTION

Crystalline lens could be replaced by intraocular lens (IOL) in cataract surgery. Since IOL was first used in transplanting in 1949, various researches have been carried out on IOL materials. As there is a great evolution on operation methods, with the advancement of operation methods, demands on the characteristics of IOL have also changed a lot. Recently, with the popularity of phacoemulsification procedure, it is possible to open a very small incision to extract opaque crystalline lens and finish the operation. So demands on characteristics of implanted IOL have changed continuously. For example, characteristics of so-called foldable IOL make it possible to implant through the small incision in foldable form and open in lens capsule. At the same time, various researches have been carried out on IOL materials. Usually, polymethyl methacrylate (PMMA), silicone, acrylic resin are widely used as the IOL materials, while polysiloxane and acrylic resin can be used as foldable IOL materials. Copolymer of ethoxyl methacrylate and methyl methacrylate may also have been used recently. On the other hand, during the studies on these kinds of material, in order to prevent from the effect of UV ray on retina, they can contain UV absorber such as hydoxylbenzophenbone, hydroxylphenyl benzotriazole and so on. Moreover, in order to raise biocompatibility and prevent from deposition of cell, polysaccharide such as heparin is coated on IOL surface in practice.

There have been reports on the polymers whose partial structure unit is acrylic acid monomer with characteristic pyrrolidone group in the present invention, especially those used in ocular lens, such as copolymer of methacryloyloxyethyl-2- pyrrolidone and acrylic acid (JP Laid-Open 28705/1992), polymer polymerized from the monomer which is formed from amidation of acrylic acid and pyrrolidone (JP Laid-Open 43208/1990), polymer of polyoxyalkylene structure with pyrrolidone group (JP Laid-Open 8218/1990) and so on. As for copolymers formed form three components, there have been reports only on copolymer of vinyl pyrrolidone, ethoxyl methacrylate and methyl methacrylate (JP Laid-Open 105250/1978), copolymer of methacryloyloxy ethyl-2-pyrrolidone, alkyl methacrylate and fluoroalkyl methacrylate (JP Laid-Open 150197/1993). Moreover, the main object of these studies is the application on soft contact lens and not on IOL. There have been no report especially on the possibility of application of foldable IOL in the invention.

### PROBLEMS TO BE SOLVED BY THE INVENTION

The object of the present invention is the application of ocular lens, in detail, the application of foldable IOL. During the studies on foldable IOL material, it is necessary to consider refractive index, tensile strength and recovery speed of the material. For example:
1) If refractive index is too low, thickness of IOL increases too much; if refractive index is too high, contraction difference of periphery becomes large. So it is necessary to choose proper refractive index. In the concrete, the preferable range is 1.4 - 1.6.
2) Because tweezers are used to implant foldable IOL, tensile strength bearing their operation is needed.
3) After foldable IOL are implanted, they must be recovered to their original form in lens capsule. Recovering speeds vary with the difference of the custom and competence among operators. The possibility of mechanical bruise of peripheral tissue usually increases with the acceleration of recovering speed. On the other hand, with the slowdown of recovering speed and the elongation of operation, the possibility of mechanical bruise of peripheral tissue increases. Therefore, for the foldable IOL, suitable recovering speed is needed.

Moreover, the characteristics of being shaped easily should be taken into consideration.

Based on these opinions, various foldable IOL can be used in practice, while it is hopeful to develop more preferable material.

### THE RESOLUTION OF THE PROBLEMS

The inventor, with the consideration of the above problems, studied on the results of more applicable material of foldable IOL and found that copolymer of hydroxyl alkyl acrylate derivative, alkyl acrylate derivative and acrylic acid derivative with pyrrolidone group could be used especially as good material.

### SUMMARY OF THE INVENTION

The present invention relates to the copolymers polymerized from the monomers represented by the following (a), (b) and (c), and the copolymers comprises the following structure units [I], [II] and [III], the process for preparing copolymers and ocular lens formed from the copolymers.
a) 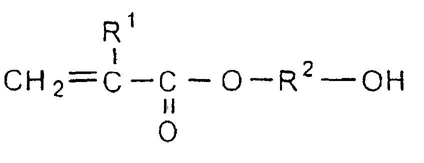 Wherein R¹ represents hydrogen atom or lower alkyl group, and R² represents lower alkylene which can be substituted by hydroxyl group, moreover, there may be oxygen atom in the alkylene chain. The followings are same.
b) 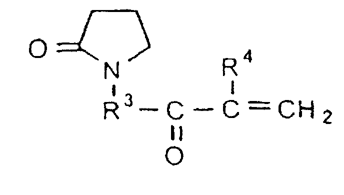 Wherein R³ represents 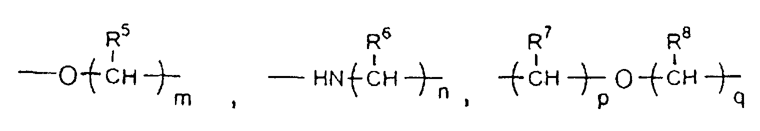 or a single bond, in which R⁵, R⁶, R⁷ and R⁸ respectively represent hydrogen atom or lower alkyl group and m, n, p and q are integers from 1 to 4, and R⁴ represents hydrogen atom or lower alkyl group. The followings are same.
c) 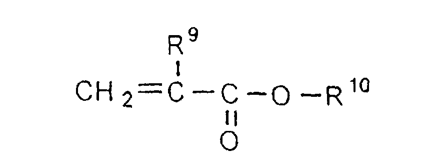
Wherein R⁹ represents hydrogen atom or lower alkyl group, R¹⁰ represents lower alkyl group. The followings are same.

When structure units are used to represent the copolymer, the copolymer is composed of the following structure units [I], [II], and [III], wherein it comprises 60-80 wt% of [I], 10-30 wt% of [II] and 5-20 wt% of [III], based on the total weight of 100 wt%.

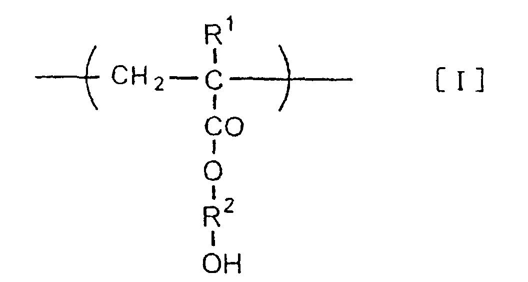

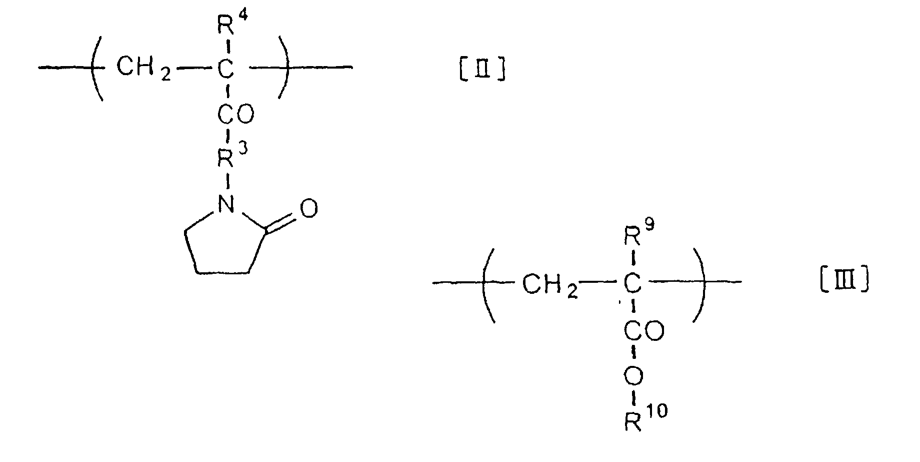

In the above formulas, said lower alkyl group is straight or branched chain alkyl groups having 1 to 6 carbon atoms such as methyl, ethyl, propyl, isopropyl, buthyl, isobutyl, hexyl; lower alkylene group is straight or branched chain alkylene groups having 1 to 6 carbon atoms such as -CH₂-, -(CH₂)₂-, -(CH₂)₃-, -CH(CH₃)₂-, -(CH₂)₄-, -(CH₂)₆-. In the above description, especially, the preferable example is that R¹ represents hydrogen atom or methyl group, R² represents -(CH₂)₂- or -(CH₂)₃-, R³ represents -OCH₂-, -OCH₂CH₂-, -NHCH₂- or a single bond, R⁴ represents hydrogen atom or methyl group, R⁹ represents hydrogen atom or methyl group and R¹⁰ represents methyl group or isobutyl group.

It is preferable to combine the above groups to use. Especially, the preferable combination is that R¹ represents methyl group, R² represents - (CH₂)₂-, R³ represents -OCH₂CH₂-, R⁴ represents methyl group, R⁹ represents methyl group and R¹⁰ represents methyl group.

About structure unit ratios of the copolymer in the present invention, preferably, the content of [I] is 65-75 wt%, the content of [II] is 15-25 wt% and the content of [III] is 5-15 wt%, based on the total weight of 100 wt%, more preferably, the content of [I] is 70 wt%, the content of [II] is 21 wt% and the content of [III] is 9 wt%.

According to the present invention, it is preferable that R¹ represents hydrogen atom or methyl group, R² represents -(CH₂)₂- or -(CH₂)₃-, R³ represents -OCH₂-, -OCH₂CH₂-, -NHCH₂- or a single bond, R⁴ represents hydrogen atom or methyl group, R⁹ represents hydrogen atom or methyl group, R¹⁰ represents methyl or isobutyl group, the content of [I] is 60-80 wt%, the content of [II] 10-30 wt% and the content of [III] 5-20 wt%, based on the total weight of 100 wt%. More preferably, R¹ represents methyl group, R² represents ethylene group, R³ represents -OCH₂CH₂-, R⁴ represents methyl group, R⁹ represents methyl group, R¹⁰ represents methyl group, the content of [I] is 70 wt%, the content of [II] is 21 wt% and the content of [III] is 9 wt%.

Monomers a), b) and c), by potassium persulfate, ammonium persulfate, benzophenone or methacryloyloxy hydroxylbenzophenone used as polymerization initiating agent, could be copolymerized to well synthesize the copolymers of the present invention.

Generally, azodiisobutyronitrile is most widely used as polymerization initiating agent in copolymer synthesis, but when it is used in synthesis of foldable IOL material which is application purpose of the present invention, mechanical strength of the material is not satisfactory. The inventor precisely studied on polymerization initiating agents and found that when potassium persulfate, ammonium persulfate, benzophenone or methacryloyloxy hydroxylbenzophenone used as polymerization initiating agent, foldable material with satisfactory mechanical strength could be obtained.

On the other hand, the molecular weight of the copolymer of the present invention (viscosity-average molecular weight) only needs to be ten thousand or over. Generally, the molecular weight has no great effect on characteristics of the copolymers, and it is not an important factor, but it has effect on strength of the copolymers. Relation between the molecular weight and strength of the copolymers is about linear proportional relation when the molecular weight is lower than ten thousand, but if the molecular weight is higher than ten thousand, strength of the copolymers attains maximum value and the value accesses constant value. Therefore, when the molecular weight is lower than ten thousand; strength of the copolymers is not stable and it is likely to be troublesome when the copolymers used as foldable IOL, but when the molecular weight is higher than ten thousand, there is no problem. However, if the molecular weight is too high, strength of the copolymers used as foldable IOL is not preferable. So the preferable range of the molecular weight of the copolymers is from ten thousand to one hundred thousand. The molecular weight of the copolymers in the following examples is also within the range.

In accordance with the composition of the present invention, through selecting properly the ratio of every component, refractive index, tensile strength, recovering speed can be designed according to needs. For example, when the ratio of structure unit [I] increases, there is almost no effect on refractive index and recovering speed, but there is a slight decreasing trend in tensile strength; when the ratio of structure unit [II] increases, refractive index increases, there is almost no effect on recovering speed, but there is a little decreasing trend in tensile strength. When the ratio of structure unit [III] increases, recovering speed and tensile strength increase, but there is a little decreasing trend in refractive index. Through good use of these characteristics, it is possible to obtain the expected foldable IOL. The preferable ratio of each structure unit is the same as the above description. If the preferable component ratio is used, refractive index is 1.4-1.6, recovering speed is not too fast or too slow, i.e. 3-6 seconds, and tensile strength is just the one which could fully bear tweezers operation, that is to say, after implantation in eyes (being swollen-wetted), foldable IOL with tensile strength being 10 psi or over could be obtained. On another occasion, according to custom of operator, recovering speed could be designed to be 3-6 seconds with 2 seconds variation amplitude.

There are hydrophilic and hydrophobic foldable IOL, and they are used respectively according to purpose. The copolymers of the present invention are hydrophilic. Hydrophilic foldable IOL would be stored under dry condition.

Before utilization, being swollen and wetted with distilled water, foldable IOL are implanted in eyes. It is possible to reckon hydrophilic extents in accordance with rate of water content. But, hydrophilic extents have effect on biological affinity and softness of foldable IOL. Under the circumstances of the present invention, it is possible to choose rate of water content by selecting ratio of every structure unit. For example, if the ratios of structure units [I] and [II] increase, rates of water content increase; if the ratio of structure unit [III] increases, rate of water content decreases. To rate of water content, the preferable range is 25-50%.

UV absorbers are widely used in IOL in order to prevent from the bad effect on retina, and the copolymers of the present invention can also contain UV absorber to some extents. There are two methods to contain UV absorber: Containing UV absorber with physical method and combining UV absorber with the copolymers with chemical method. In the present invention, the two methods could also be used. Especially with the chemical method, there is an advantage that UV absorber would not be left out from IOL when IOL are used.

The chemical combination is that UV absorber itself which has polymerizable double bond, may be copolymerized with the above monomers to form the copolymers. The content of UV absorber is very little to the total weight, and it has almost no effect on characteristics of the copolymers. But if the content of UV absorber is too much, the copolymers become brittle. The preferable range of content of UV absorber is 0.5-2.0 wt%, and the more preferable range is 0.8-1.5 wt%.

There is no special limits on the varieties of UV absorbers, so long as used in IOL. For example, benzophenone, benzotriazole series or their acrylic acid derivatives, in the concrete, hydroxylbenzophenone, hydroxylbenzotriazole or their acrylic acid derivatives such as 4-methacryloyloxy-2-hydroxylbenzophenone are used as UV absorbers.

The copolymers of the present invention can be cross linked between molecules. Physical strength and rate of water content can be adjusted through cross-linking. Compounds of acrylate or acrylamide series can be used as cross-linking agent, for example, ethylene glycol bismethacrylate, diethylene glycol bismethacrylate or N, N'-methylene bisacrylamide. When content of cross-linking agent is too much, the copolymers would become hard and recovering speed would become fast, however, the copolymers become brittle. The preferable range of content of cross-linking agent is 0.01-2.0 wt%, the more preferable range is 0.1-1.5 wt% and the most preferable range is 0.5-1.0 wt%.

In order to raise biocompatibility and prevent from deposition of cell, the technique of polysaccharide coating on IOL surface has been used recently. Because there are free hydroxyl groups on the terminals of the copolymers of the present invention, which can combine with polysaccharide by covalent bond, separation of the coatings doesn't occur. Moreover, it is possible to make covalent bond formed easily. For example, divinyl sulfone could do the job. Preferably, polysaccharide is heparin, hyaluronic acid or its salt such as sodium salt, potassium salt and so on.

Moreover, as a characteristic of the copolymers of the present invention, the value of monomer reactivity ratio r₁ × r₂ (MRR) of acrylic acid derivatives with pyrrolidone group, that is to say, components b) and a), accesses 1.0. The value of r₁ × r₂ accessing 1.0 means ideal polymerization. If component a) is 2-hydroxylethyl methacrylate and component b) is N-pyrrolidonoethyl acrylate, the value of r₁ × r₂ is 1.03. Testing method of MRR is in accordance with Mayo-Lewis Method (J. Am. Chem. Soc., 1994, 66, 1594).

The present invention relates to IOL material having flexible and soft property. After an operation with tweezers, marks of tweezers remains and it must take some time to recover to its original form. But the problem could be resolved because the copolymers of the present invention have good recovering ability.

After implantation of usual silicone series IOL, if laser is used to cure secondary cataract, crystalline lens often become opaque and transparency would be damaged. On another occasion, there may be slight denaturation on periphery, then visual field would be damaged to some extents. However, when the copolymers of the present invention are used, opaque would not be found, transparency would be maintained and there would be no denaturation on periphery. The copolymers of the present invention could form easily applied foldable IOL. The shaping method has been reported in public. The copolymers of the present invention are especially preferable for foldable IOL, and they are also preferable to be used on soft contact lens.

The present invention is further explained by the following examples of its practice.

### REFERENCE EXAMPLE (SYNTHESIS OF MONOMER)

### REFERENCE EXAMPLE 1

### Synthesis of 2-(2-pyrrolidone-1-yl) ethyl acrylate(PyEA)

Into the solution of 1-(2-hydroxyethyl)-2-pyrrolidone (50 ml) and triethylamine (85 ml) dissolved in chloroform (150 ml), the solution of acryloyl chloride (50 ml) dissolved in chloroform (100 ml) was dropped in two hours with stirring. The reactive solution was stirred at 4°C for 20 hours, then at 50°C for 2 hours, after cooling, the solution was filtered. The filtrate was washed with 15% of sodium carbonate aqueous solution, then concentrated under vacuum. The obtained oily material was distillated under vacuum. The yield of the title compound is 70%.
bp: 112- 113°C/0.5 Torr
NMR (ppm, TMS, CDCl₃): 6.14 (m, 3H), 5.80 (s, I H), 4.30 (t, 2H), 3.60 (m, 4H), 2.20 (m, 4H)
IR(KBr,cm⁻¹): 1735, 1676, 1639, 1361

With the similar synthesis method to described in Example 1, the following compounds were synthesized.
2-(2-pyrrolidone-1-yl) ethyl methacrylate (PyEMA)
   bp: 120- 122°C/0.5 Torr
   NMR (ppm, TMS, CDCl₃): 6.10 (s, 3H), 5.59 (s, 1H), 4.29 (t, 2H), 3.59 (m, 4H), 2.30 (m, 4H), 1.94 (m, 3H)
   IR (KBr, cm⁻¹): 1735, 1676, 1639, 1361
2-pyrrolidone-1-ylmethyl acrylate (PyMA)
   bp: 92 - 93°C/0.5 Torr
   NMR (ppm, TMS, CDCl₃): 6.34-6.83 (m, 2H),
   6.06-6.12 (m, 1H), 5.37 (m, 2H), 3.57-3.69 (m, 2H),
   1.91-2.50 (m, 4H)
   IR (KBr, cm⁻¹): 1708, 1639, 1415
2-pyrrolidone-1-ylmethyl methacrylate (PyMMA)
   bp: 95-96°C/0.5 Torr
   NMR (ppm. TMS, CDCl₃): 6.13-6.16 (s, 1H),
   5.59-5.61 (m, 1H), 5.41 (s, 2H), 3.55 (t, 2H), 1.9-2.43 (m, 4H), 1.96(m, 3H)
   IR (KBr, cm⁻¹): 1716, 1636, 1420

### REFERENCE EXAMPLE 2

### Synthesis of N-(2-pyrrolidone-1-ylmethyl)acrylamide (PyMAm)

The mixture of 1-methoxy methyl-2-pyrrolidone (65.6 g), acrylamide (75.5 g), toluene sulfonic acid (0.20 g) and phenothiazine (0.20 g) was stirred under the condition of N₂ for 1 hour at 150°C while removing methanol by distillation. The residue was cooled, then recrystallized in acetone to give the title compound in the yield of 40%.
mp: 73-74°C
NMR (ppm, TMS, CDCl₃): 7.31 (m, 1H), 6.19-6.31 (m, 2H),
5.56-5.72 (m, 1H), 4.75-4.81 (d, 2H), 3.50-3.66 (t, 2H), 1.91-2.44 (m, 4H)
IR (KBr, cm⁻¹): 3440, 1680, 1200-1600

With the similar synthesis method to described in Example 2, the following compounds were synthesized.
N-(2-pyrrolidone-1-ylmethyl) methacrylamide (PyMMAm)
mp: 101-102°C
NMR (ppm. TMS, CDCl₃): 6.70 (s, 1H), 5.69 (s, 1H), 5.35 (s, 1H), 4.70-4.80 (d, 2H), 3.45-3.62 (t, 2H), 1.95-2.45 (m, 7H)

### REFERENCE EXAMPLE 3

### Synthesis of 1-acryloyl-2-pyrrolidone (NAPy)

2-pyrrolidone and acryloyl chloride were used to synthesize the title compound with the similar synthesis method to described in Example 1. The yield of title compound was 70%.
bp: 85-86°C/0.5 Torr
NMR (ppm. TMS, CDCl₃): 7.31-7.65 (m, 1H), 6.56-6.59 (m, 1H), 5.74-5.96 (m, 1H), 3.77-3.96 (m, 2H), 2.52-2.73 (m, 2H), 1.85-2.24 (2m, 2H)
IR (KBr, cm⁻¹): 1737, 1679, 1408

With the similar synthesis method to described in Example 3, the following compounds were synthesized.
1-methacroyl-2-pyrrolidone (NMAPy)
bp: 88-89°C/0.5 Torr
NMR (ppm. TMS, CDCl₃): 5.28-5.34 (s, 2H), 3.72-3.90 (m, 2H), 2.57-2.67 (m, 2H), 2.00-2.16 (m, 2H), 1.97-1.99 (s, 3H)
IR (KBr, cm⁻¹): 1745, 1676, 1403

### EXAMPLES

### Synthesis of copolymers and preparation of the sheet formed from the copolymers

### EXAMPLE 1

### Synthesis of copolymer (HEMA-PyEMA-MMA) of 2-hydroxyethyl methacrylate (HEMA), 2-(2-pyrrolidone-1-yl)ethyl methacrylate (PyEMA) and metyl methacrylate (MMA), and preparation of the sheet formed from the copolymer

Into the mixture of HEMA, PyEMA and MMA with mixing volume ratio 70:20:10 (weight ratio 70:21:9), 0.2 wt% of potassium persulfate and 10 wt% of water were added, then the mixture was added to the 0.5 mm or 0.1 mm inter space of separators and the spacer between two glass plates fixed with holder. The size of the glass plates is 6 cm × 5 cm, and they were treated with sealing agent of siloxane series and were fixed by attachment clip. The mixture was free radical-polymerized at 60°C for 22 hours, then at 90°C for 2 hours, then the mixture was treated and the polymerization was finished. After those, the sheet was taken off from the glass plates, dipped into distilled water to remove unreacted monomers, then the copolymer sheet as purpose was obtained. Moreover, size of the spacer between the two glass plates could be chosen according to needs.

With the similar preparation to described in Example 1, the following copolymers and their sheets were obtained.
HEMA-PyEA-MMA, HEMA-PyMA-MMA, HEMA-PyMMA-MMA, HEMA-NAPy-MMA, HEMA-PyMAm-MMA, HEMA-PyMMAm-MMA, HEA-PyEA-MA, HEA-PyMAm-MA, HPMA-PyEA-MMA, HEMA-PyE.

The above abbreviations represent the following compounds.
HEMA: 2-hydroxylethyl methacrylate
HEA: 2-hydroxylethyl acrylate
HPMA: 2-hydroxylpropyl methacrylate
NAPy: 1-acryloyloxy-2-pyrrolidone
PyEA: 2-(2-pyrrolidone-1-yl) ethyl acrylate
PyEMA: 2-(2-pyrrolidone-1-yl) ethyl methacrylate
Py MA: 2-pyrrolidone-1-yl methyl acrylate
PyMMA: 2-pyrrolidone-1-ylmethyl methacrylate
PyMAm; N-(2-pyrrolidone-1-ylmethyl)acrylamide
PyMMAm: N-(2-pyrrolidone-1-yl-methyl) methacrylamide
MMA: methyl methacrylate
MA: methyl acrylate
i-BuMA: isobutyl methacrylate
NMAPy: 1-methacryloyl-2-pyrrolidone

The copolymers and their sheets could be prepared with the similar method to described in the following Example 2.

### EXAMPLE 2

### Synthesis of copolymers with photopolymerizaiton and preparation of their sheets

Into the mixture of HEMA, PyEMA and MMA with mixing volume ratio 70:20:10 (weight ratio 70:21:9), 2 wt% of benzophenone or 4-methacryloyloxy-2-hydroxylbenzophenone and 0.4 wt% of N, N'-dimethylaminoethyl methacrylate were added, then the mixture was added into the 0.5 mm or 0.1 mm inter space of separators and the spacer between two glass plates fixed with holder. The size of the glass plates is 6 cm × 5 cm. They were treated with sealing agent of siloxane series and were fixed by attachment clip. The mixture was exposed under UV light of 80w mercury lamp for 48 hours to be photopolymerized. Then the sheet was taken off from the glass plates, dipped into distilled water to remove unreacted monomers, then the copolymer sheet as purpose was obtained. Moreover, the size of the spacer between the two glass plates, could be chosen according to needs.

### EXAMPLE 3

### Synthesis of the cross linked copolymers and preparation of their sheet

In Example 1, into the mixture before radical polymerization, 1.0 wt% of ethyleneglycol bismethacrylate (EGMA) was added. The following procedures were similar as that of Example 1, then the cross linked copolymer and its sheet were obtained.

In stead of EGMA, bismethacrylate such as diethyleneglycol bismethacrylate (DEGMA). bisacrylamide such as N, N'-methylene bisacrylamide can be used. On another occasion, content of cross-linking agent could be chosen according to needs.

Physiochemical properties of the copolymers sheets in Examples 1 or 3 are shown in table 1. In the table, the copolymers marked with asterisk (*) were synthesized with methods of Example 3. Others were synthesized with methods of Example 1 and Example 2, the synthetic method is photopolymerization, but physiochemical properties of the obtained copolymer are same as those of the copolymer of Example 1.

**Table 1**

| Copolymer | Rate of water content | Refractive index | Tensile strength strength | | Recovering speed |
|---|---|---|---|---|---|
| | | | wet | dry | |
| HEMA-PyEA-MMA | 34 | 1.45 | 29.2 | 1,000 | Qualified |
| HEMA-PyEMA-MMA | 31 | 1.45 | 32.1 | 1,050 | Qualified |
| HEMA-PyMA-MMA | 33 | 1.45 | 82.3 | 2,313 | Qualified |
| HEMA-PyMMA-MMA | 28 | 1.44 | 193.6 | 806 | Qualified |
| HEMA-NAPy-MMA | 33 | 1.46 | 58.1 | 2,389 | Qualified |
| HEMA-PyMAm-MMA* | 40 | 1.44 | 41.4 | - | Qualified |
| HEMA-PyMMAm-MMA* | 36 | 1.45 | 65.9 | - | Qualified |
| HPMA-PyEA-MMA* | 40 | 1.43 | 19.2 | - | Qualified |
| HEMA-PyEA-i-BuMA* | 31 | 1.45 | 51.3 | - | Qualified |

| | | | | | |
|---|---|---|---|---|---|
| *Cross-linked copolymers synthesized with method of Example 3. | | | | | |

Physiochemical properties in Table 1 were tested with the following methods.

Rate of water content: Copolymer sheet cut in disc form was added into water at 0°C for more than 2 days. After water content was in equilibrium, water on the surface was wiped out and weighed the sheet weight, that is W₁. Then the copolymer sheet was dehydrated under vacuum at 60°C for 48 hours. The weight of the dry copolymer was W₂.

Rate of water content is calculated with (W₁-W₂)/W₁.

Refractive index: Tested with Abbe refractive machine.

Tensile strength: Tested at 12cm/min tensile speed with YQ-Z-7 equipment. Unit is represented with psi. The so-called "wet" represents equilibrium state when the sheet being swollen and wetted by water and "dry" represents the state when the sheet was prepared.

Recovering speed: Sheet in equilibrium state when being swollen and wetted by water was cut in the size 3.0 × 3.0 cm. After it was folded by tweezers, time of recovering to original state was tested. The applicable time is about 3-6 seconds with 2 seconds variation amplitude and as long as the time is in the range of 3-6 seconds, it is called "qualified."

### EXAMPLE 4

### Synthesis of the copolymer with UV absorber and its sheet

In Example 1, into the mixture before radical polymerization, 0.8-1.5 wt% of 4-methacryloxy-2-hydroxylbenzophenone (MAHBP) was added and followed by the similar procedure to Example 1, the copolymer combined with UV absorber in the form of chemical bond and its sheet were obtained.

When UV spectrum of the sheet was tested with Shimazu UV-250 machine, it was found that the sheet had good absorption under 400 nm. Absorption range could change through selecting UV absorber.

### EXAMPLE 5

### Treating of sheet surface with polysaccharide

The copolymer sheet prepared in Example 1 was added into 5% of heparin aqueous solution and stored for 24 hours, then exposed in air for 1 hour. After those, the sheet was dipped into sodium carbonate buffer solution (pH 11) with 0.1% of diethyl sul fone at 40°C, then the sheet was washed with phosphoric acid buffer solution, and washed fully with water. In the sheet, heparin was combined with hydroxyl group in state of covalent bond.

Sodium hyaluronate could be used as polysaccharide to replace heparin, and the similar sheet could be obtained.

### REFERENCE EXAMPLE 4

### Testing of monomer reactivity ratio (MRR)

Testing monomer reactivity ratio (MRR) of acrylic acid derivative with pyrrolidone group which has the characteristic of monomer component of the copolymers in the present invention, such as PyEA, and the corresponding polymerizable monomer such as HEMA.

PyEA whose content varied in the range of 1 to 10 parts was mixed with one part HEMA, then azodiisobutyronitrile was added as polymerization initiating agent, and the mixture was placed into a sealed glass container with a gas outlet. The mixture was treated at 60°C for 10-45 minutes to control the content of polymer below 10%. After reaction, the copolymer was precipitated in petroleum ether. Based on the analysis of nitrogen content, content of PyEA in the copolymer was determined. In accordance with Mayo-Lewis Method (J. Am. Chem. Soc., 1994, 66, 1594), reactive ratio r₁ and r₂ of PyEA/HEMA was determined. The value of r₁ is 0.43, the value of r₂ is 2.40 and the value of monomer reactivity ratio r₁ × r₂ is 1.03.

### THE EFFECTS OF THE PRESENT INVENTION

The present invention supplies novel copolymer useful for ocular lens, especially for foldable IOL.

The advantages of copolymers are shown as the following:
1. Exhibit fast recovering property from fold state.
2. Exhibit good refractive index.
3. Good physical strength (tensile strength).
4. Expected property could be obtained through changing composition ratio of each monomer.
5. There would be no trace, even if tweezers are used to operate.
6. Opaque would not occur even if irradiated with laser, and transparency could be maintained.
7. The copolymer could be hydrated after synthesis of the copolymer because there are hydroxyl groups in molecules.
8. The surface of copolymers could easily be treated with polysaccharide, and the copolymers could be combined with polysaccharide in covalent bond state, so it is hardly to be isolated from each other.
9. It is easy to cross link between molecules.
10. The value of monomer reactivity ratio (MRR) r₁ × r₂ of acrylic acid derivative with pyrrolidone group which has the characteristic of monomer component of the copolymers in the present invention, that is to say, components b) and a), accesses 1.0. The value of r₁ × r₂ accessing 1.0 means that the polymerization is ideal.

## Claims

1. A copolymer, **characterized in that** the copolymer is composed of the following structure units [I], [II] and [III], wherein it comprises 60 - 80 wt % of [I], 10 - 30 wt % of [II] and 5 - 20 wt % of [III], based on the total weight of 100%, 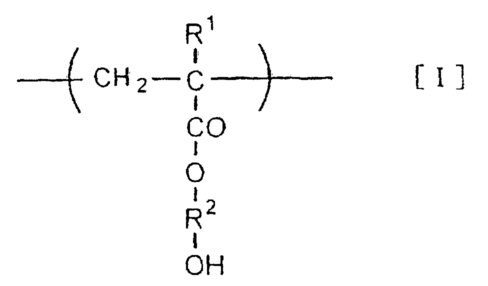 wherein R¹ represents hydrogen atom or lower alkyl group, R² represents lower alkylene group and the lower alkylene group can be substituted by hydroxyl group, and there can be oxygen atom in the lower alkylene group chain, 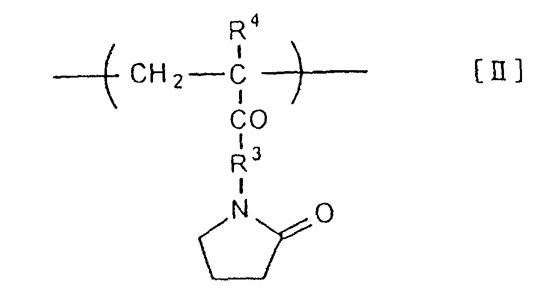 wherein R³ represents 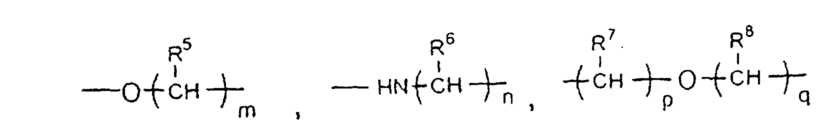 or single bond, in which R⁵, R⁶, R⁷and R⁸ respectively represent hydrogen atom or lower alkyl group and m, n, p and q are integers from 1 to 4, and R⁴ represents hydrogen atom or lower alkyl group, 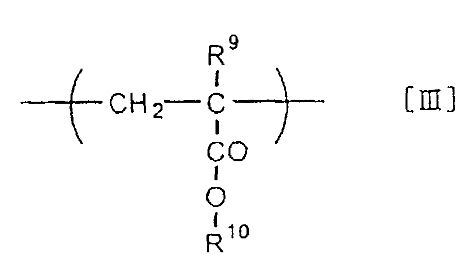 wherein R⁹ represents hydrogen atom or lower alkyl group and R¹⁰ represents lower alkyl group, said lower alkyl or alkylene groups having 1 to 6 carbon atoms.

2. The copolymer as claimed in claim 1, wherein said molecular weight is from ten thousand to one hundred thousand.

3. The copolymer as claimed in claim 1, wherein said R¹ represents hydrogen atom or methyl group and R² represents -(CH₂)₂- or -(CH₂)₃-.

4. The copolymer as claimed in claim 1, wherein said R₃ represents - OCH₂-, -OCH₂CH₂-, -NHCH₂- or a single bond and R⁴ represents hydrogen atom or methyl group.

5. The copolymer as claimed in claim 1, wherein said R⁹ represents hydrogen atom or methyl group and R¹⁰ represents methyl or isobutyl group.

6. The copolymer as claimed in claim 1, wherein said R¹ represents hydrogen atom or methyl group and R² represents -(CH₂)₂- or -(CH₂)₃-, R³ represents -OCH₂-, -OCH₂CH₂-, -NHCH₂- or a single bond, R⁴ represents hydrogen atom or methyl group, R⁹ represents hydrogen atom or methyl group and R¹⁰ represents methyl or isobutyl group.

7. The copolymer as claimed in claim 1, wherein said R¹ represents methyl group, R² represents -(CH₂)₂-, R³ represents -OCH₂CH₂-, R⁴ represents methyl group, R⁹ represents methyl group and R¹⁰ represents methyl group.

8. The copolymer as claimed in claim 1, wherein said content of [I] is 65 - 75 wt %, content of [II] is 15 - 25 wt % and content of [III] is 5 - 15 wt %, based on the total weight of 100 wt %.

9. The copolymer as claimed in claim 1, wherein said content of [I] is 70 wt %, content of [II] is 21 wt % and content of [III] is 9 wt %.

10. A copolymer as claimed in claim 1, **characterized in that** the copolymer is composed of the following structure units [I], [II] and [III], wherein it comprises 60 - 80 wt % of [I], 10-30 wt % of [II] and 5 - 20 wt % of [III], based on the total weight of 100 wt %, wherein said R¹ represents hydrogen atom or methyl group, R² represents -(CH₂)₂-, or -(CH₂)₃-, R³ represents -OCH₂-, - OCH₂CH₂-, -NHCH₂- or a single bond, R⁴ represents hydrogen atom or methyl group, R⁹ represents hydrogen atom or methyl group and R¹⁰ represents methyl or isobutyl group.

11. The copolymer as claimed in claim 1, wherein said content of [I] is 70 wt %, content of [II] is 21 wt % and content of [III] is 9 wt % and R¹ represents methyl group, R² represents ethylene group, R³ represents - OCH₂CH₂-, R⁴ represents methyl group, R⁹ represents methyl group and R¹⁰ represents methyl group.

12. The copolymer as claimed in claim 1, wherein UV absorber is contained.

13. The copolymer as claimed in claim 12, **characterized in that** containing form of UV absorber is chemical combination with the copolymer.

14. The copolymer as claimed in claim 13, wherein said content of UV absorber is 0.5 - 2.0 wt % of the copolymer.

15. The copolymer as claimed in claim 13, wherein said content of UV absorber is 0.8 - 1.5 wt % of the copolymer.

16. The copolymer as claimed in claim 12, wherein said UV absorber is hydroxyl benzophenone.

17. The copolymer as claimed in claim 12, wherein said UV absorber is one of compounds of acrylic acid series with hydroxylbenzophenone or benzotriazole residual group.

18. The copolymer as claimed in claim 1 or 12, wherein it is cross linked with said cross-linking agent of compounds of acrylate series or acrylamide series.

19. The copolymer as claimed in claim 18, wherein said cross-linking agent is selected from ethylene glycol bismethacrylate, diethylene glycol bismethacrylate or N,N'-methylene bisacrylamide and the content of cross-linking agent is 0.01-2.0 wt % of the copolymer.

20. The copolymer as claimed in claim 18, wherein said content of cross-linking agent is 0.1 - 1.5 wt% of the copolymer.

21. The copolymer as claimed in claim 18, wherein said content of cross-linking agent is 0.5 - 1.0 wt % of the copolymer.

22. The copolymer as claimed in any of claim 1, 2 or 18, wherein said polysaccharide is used to treat the surface of copolymer.

23. The copolymer as claimed in claim 22, wherein said surface treating is achieved by the covalent bond between the copolymer and polysaccharide.

24. The copolymer as claimed in claim 22, wherein said covalent bond is formed by using divinyl sulfone.

25. The copolymer as claimed in claim 22, wherein said polysaccharide is heparin, hyaluronic acid or its salt.

26. A ocular lens which is formed from the copolymer as claimed in any of claims 1, 12, 18 and 22.

27. A foldable ocular lens which is formed from the copolymer as claimed in any of claims 1, 12, 18 and 22.

28. A foldable ocular lens which is formed from the copolymer as claimed in any of claims 1, 12, 18 and 22 and has the following characteristics:
a) refractive index is 1.4 - 1.6;
b) recovering speed from fold state to original state is 3-6 seconds;
c) when being swollen-wetted, tensile strength is over 10 psi (6.895 x 10⁴ Pa).

29. A foldable ocular lens which is formed from the copolymer as claimed in claims 1, wherein said UV absorber is contained, cross-linking agent is used to cross link the copolymer and polysaccharide is used to treat the surface of copolymer.

30. The foldable ocular lens as claimed in claim 29, wherein said UV absorber is selected from any kinds of compounds of benzophenone series, compounds of benzotriazole series or compounds of acrylic acid series with hydroxylbenzophenone or benzotriazole residual group, cross-linking agent is selected from compounds of acrylate series or compounds of acrylamide series and polysaccharide is selected form heparin, hyaluronic acid or its salt.

31. A copolymer which is copolymerized from the following a), b) and c):
a) 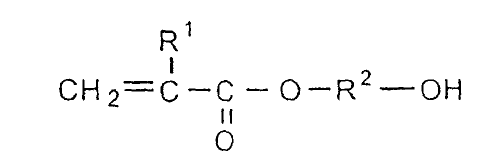 wherein R¹ represents hydrogen atom or lower alkyl group, R² represents lower alkylene group, which can be substituted by hydroxyl group, and there can be oxygen atom in the alkylene group chain,
b) 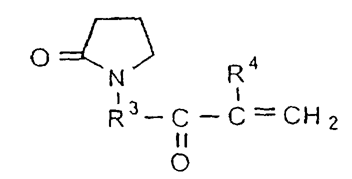 wherein R³ represents 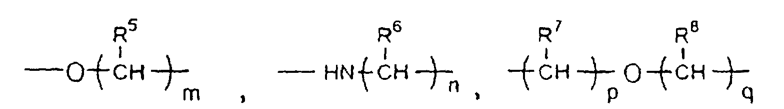 or a single bond, in which R⁵, R⁶, R⁷ and R⁸ respectively represent hydrogen atom or lower alkyl group and m, n, p and q are integers from 1 to 4, and R⁴ represents hydrogen atom or lower alkyl group,
c) 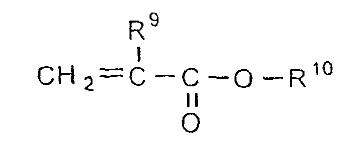
wherein R⁹ represents hydrogen atom of lower alkyl group and R¹⁰ represents lower alkyl group, said lower alkyl or alkylene groups having 1 to 6 carbon atoms.

32. The copolymer as claimed in claim 31, wherein the molecular weight is from ten thousand to one hundred thousand.

33. A process for preparing a copolymer as claimed in claim 31,
**characterized in that** components a), b) and c) as claimed in claim 31 are polymerized with polymerization initiating agent such as potassium persulfate, ammonium persulfate, benzophenone or methacryloyloxy hydroxylbenzophenone.

## Patentansprüche

1. Ein Copolymer, **dadurch gekennzeichnet, daß** das Copolymer sich aus den folgenden Struktureinheiten [I], [II] und [III] zusammensetzt, wobei es, basierend auf dem absoluten Gewicht von 100 %, 60 bis 80 Gew.% von [I], 10 bis 30 Gew.% von [II] und 5 bis 20 Gew.% von [III] enthält, 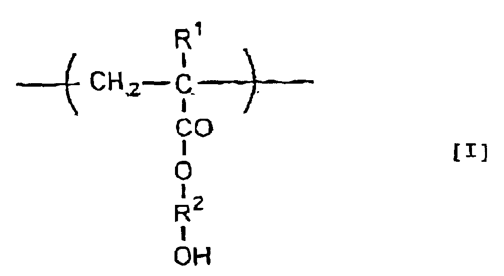 wobei R¹ ein Wasserstoffatom oder eine niedere Akylgruppe repräsentiert, R² eine niedere Alkylengruppe repräsentiert, die niedere Alclylengruppe durch eine Hydroxylgruppe substituiert sein kann und Sauerstoff in der Kette der niederen Alkylengruppe vorhanden sein kann 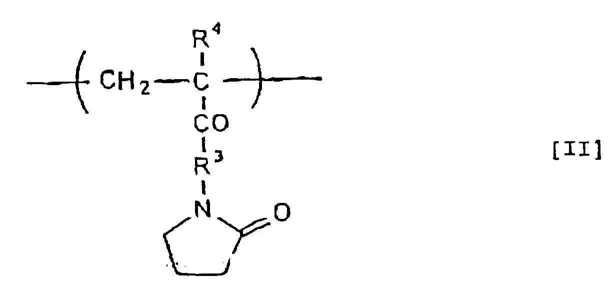 wobei R³ 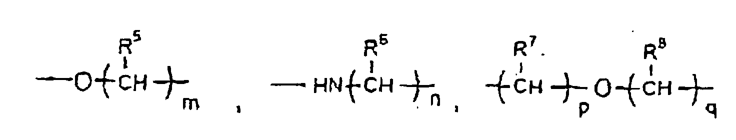 oder eine Einfachbindung repräsentiert, R⁵, R⁶, R⁷, R⁸ entsprechend Wasserstoff oder eine niedere Alkylgruppe repräsentieren und m, n, p und q zahlen von 1 bis 4 sind, und R⁴ Wasserstoff oder eine niedere Alkylgruppe repräsentiert, 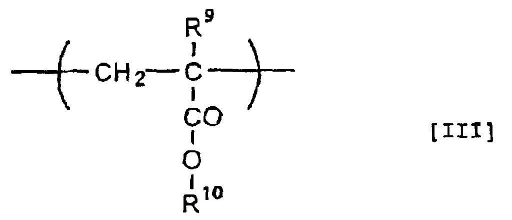 wobei R⁹ ein Wasserstoffatom oder eine niedere Alkylgruppe sowie R¹⁰ eine niedere Alkylgruppe, repräsentiert und die genannten niederen Alkylgruppen oder Alkylengruppen 1 bis 6 Kohlenstoffatome aufweisen.

2. Copolymer nach Anspruch 1, **dadurch gekennzeichnet, daß** das Molekulargewicht zwischen 10 000 und 100 000 beträgt.

3. Copolymer nach Anspruch 1, **dadurch gekennzeichnet, daß** R¹ ein wasserstoffatom oder eine Methylgruppe und R² -CH₂)₂- oder -CH₂)₃-, repräsentiert.

4. copolymer nach Anspruch 1, **dadurch gekennzeichnet, daß** R³ -OCH₂-, -OCH₂CH₂-, -NHCH₂- oder eine Einfachbindung und R⁴ ein Wasserstoffatom oder eine Methylgruppe repräsentiert.

5. Copolymer nach Anspruch 1, **dadurch gekennzeichnet, daß** R⁹ ein Wasserstoffatom oder eine Methylgruppe und R¹⁰ eine Methylgruppe oder isobutylgruppe repräsentiert.

6. Copolymer nach Anspruch 1, **dadurch gekennzeichnet, daß** R¹ ein Wasserstoffatom oder eine Methylgruppe repräsentiert, R² -(CH₂)₂- oder -(CH₂)₃- repräsentiert, R³ -OCH₂-, -OCH₂CH₂-, -NHCH₂- oder eine Einfachbindung repräsentiert, R⁴ ein Wasserstoffatom oder eine Methylgruppe repräsentiert, R⁹ ein Wasserstoffatom oder eine Methylgruppe repräsentiert und R¹⁰ eine Methylgruppe oder Isobutylgruppe repräsentiert.

7. Copolymer nach Anspruch 1, **dadurch gekennzeichnet, daß** R¹ eine Methylgruppe repräsentiert, R² -(CH₂)₂repräsentiert, R³ -OCH₂CH₂- repräsentiert, R⁴ eine Methylgruppe repräsentiert, R⁹ eine Methylgruppe repräsentiert und R¹⁰ eine Methylgruppe repräsentiert.

8. Copolymer nach Anspruch 1, **dadurch gekennzeichnet, daß** der Anteil von [I] 65 bis 75 Gew.%, der Anteil von [II] 15 bis 25 Gew.% und der Anteil von [III] 5 bis 15 Gew.%, basierend auf dem absoluten Gewicht von 100 %, beträgt.

9. Copolymer nach Anspruch 1, **dadurch gekennzeichnet, daß** der Anteil von [I] 70 Gew.%, der Anteil von [II] 21 Gew.% und der Anteil von [III] 9 Gew.% beträgt.

10. Copolymer nach Anspruch 1, **dadurch gekennzeichnet, daß** sich das Copolymer aus den Struktureinbeiten [I], [II] und [III] zusammensetzt, wobei es, basierend auf dem absoluten Gewicht von 100 Gew.%, 60 bis 80 Gew.% [I], 10 bis 30 Gew.% [II] und 5 bis 20 Gew.% [III] enthält, wobei R¹ ein Wasserstoffatom oder eine Methylgruppe repräsentiert, R² -(CH₂)₂- oder -(CH₂)₃- repräsentiert, R³ -OCH₂-, -OCH₂CH₂-, -NHCH₂oder eine Einfachbindung repräsentiert. R⁹ Wasserstoff oder eine Methylgruppe repräsentiert und R¹⁰ eine Methylgruppe oder isobutylgruppe repräsentiert.

11. Copolymer nach Anspruch 1, **dadurch gekennzeichnet, daß** der Anteil von [I] 70 Gew.%, der Anteil von [II] 21 Gew.% und der Anteil von [III] 9 Gew.% beträgt, und R¹ eine Methylgruppe, R² eine Ethylengruppe, R³ -OCH₂CH₂-, R⁴ eine Methylgruppe, R⁹ eine Methylgruppe und R¹⁰ eine Methylgruppe repräsentiert.

12. Copolymer nach Anspruch 1, **dadurch gekennzeichnet, daß** ein UV-Absorber enthalten ist.

13. Copvlymer nach Anspruch 12, **dadurch gekennzeichnet, daß** die enthaltene Form des UV-Absorbers eine chemische Verbindung mit dem Copolymer ist.

14. Copolymar nach Anspruch 13, **dadurch gekennzeichnet, daß** bezogen auf das Copolymer der Anteil des UV-Absorbers 0,5 bis 2,0 Gew.% beträgt.

15. Copolymer nach Anspruch 13, **dadurch gekennzeichnet, daß** der Anteil des UV-Absorbers bezogen auf das Copolymer 0,8 bis 1,5 Gew,% beträgt.

16. Copolymer nach Anspruch 12, **dadurch gekennzeichnet, daß** der UV-Absorber Hydroxylbenzophenon ist.

17. Copolymer nach Anspruch 12, **dadurch gekennzeichnet, daß** der UV-Absorber eine Verbindung aus der Gruppe der Acrylsäure Abkömmlinge mit Bydroxylbenzophenon- oder Benzotriazolrest ist.

18. Copolymer nach Anspruch 1 oder 12, **dadurch gekennzeichnet, daß** das Copolymer mit Acrylat-Derivaten oder Acrylamid-Derivaten als vernetzungsreagenz vernetzt wird.

19. Copolymer nach Anspruch 18, **dadurch gekennzeichnet, daß** das Vernetzungsreagenz aus der Gruppe Ethylenglykolbismethacrylat, Diethylenglykolbismethacrylat, N,N'-Methylenbisacrylamid ausgewählt wird, und der Anteil des Vernetzungsreagenzes 0,01 bis 2,0 Gew.%, bezogen auf das Copolymer, beträgt.

20. Copolymer nach Anspruch 18, **dadurch gekennzeichnet, daß** der Anteil des Vernetzungsreagenzes 0,1 bis 1,5 Gew.%, bezogen auf das Copolymer, beträgt.

21. Copolymer nach Anspruch 18, **dadurch gekennzeichnet, daß** der Anteil des vernetzungsreagenzes 0,5 bis 1,0 Gew.%, bezogen auf das Copolymer, beträgt.

22. Copolymer nach einem der Ansprüche 1, 2 oder 18, **dadurch gekennzeichnet, daß** Polysaccharide zur Behandlang der Oberfläche des Copolymers eingesetzt werden.

23. copolymer nach Anspruch 22, **dadurch gekennzeichnet, daß** die Oberflächenbehandlung durch eine kovalente Bindung zwischen Copolymer und Polysaccharid erreicht wird.

24. Copolymer nach Anspruch 22, **dadurch gekennzeichnet, daß** die kovalente Bindung durch den Einsatz von Divinylsulfon erzeugt wird.

25. Copolymer nach Anspruch 22, **dadurch gekennzeichnet, daß** Polysaccharid Heparin, Hyaluronsäure oder deren Salz ist.

26. Eine okulare Linse, die aus Copolymeren nach einem der Ansprüche 1, 12, 18 und 22 gebildet ist.

27. Eine faltbare okulare Linse, die aus Copolymeren nach einem der Ansprüche 1,12, 18 und 22 gebildet ist.

28. Eine faltbare okulare Linse, die aus Copolymeren nach einem der Ansprüche 1, 12, 18 und 22 gebildet ist und folgende Eigenschaften aufweist:
a) der Brechungsindex beträgt 1,4 bis 1,6;
b) die Rückfaltungsgeschwindigkeit vom gefalteten in den ursprünglichen zustand beträgt 3 bis 6 Sekunden;
c) im gequollenen und befeuchteten Zustand ist die Zugfestigkeit größer als 10 psi (6,895 x 10⁴ Pa).

29. Eine faltbare okulare Linse, die aus Copolymer nach Anspruch 1 gebildet ist, **dadurch gekennzeichnet, daß** UV-Absorber enthalten ist, ein Vernetzungsreagenz zur Vernetzung des Copolymers eingesetzt wird und Polysaccharide zur Oberflächenbehandlung des Copolymers eingesetzt werden.

30. Faltbare okulare Linse nach Anspruch 29, **dadurch gekennzeichnet, daß** der UV-Absorber ausgewählt ist aus der Gruppe Benzophenon-Derivate, Verbindungen von Benzotriazol-Derivaten, Verbindungen von Acrylsäure-Derivaten mit Hydroxylbenzophenon- oder Benzotriazolresten, das Vernetzungsreagenz ausgewählt ist aus der Gruppe Verbindungen von Acrylat-Derivaten, Verbindungen von Acrylamid-Derivaten und Polysaccharide ausgewählt sind aus der Gruppe Heparin, Hyaluronsäure, Salz der Hyaluronsäure.

31. Copolymer, das durch Copolymerisation der folgenden Monomeren a), b) und c) erhalten wird:
a) 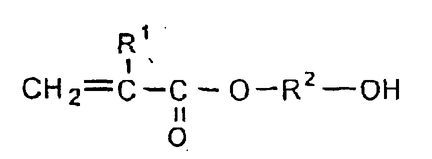 wobei R¹ ein Wasserstoffatom oder eine niedere Alkylgruppe repräsentiert, R² eine niedere Alkylengruppe repräsentiert, die durch eine Hydroxylgruppe substituiert sein kann und Sauerstoff in der Kette der Alkylengruppe enthalten sein kann,
b) 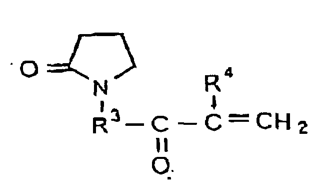 wobei R³ 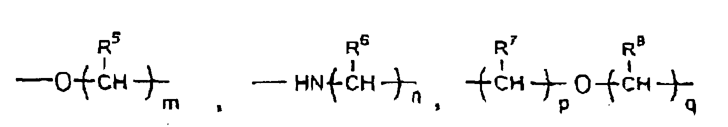 oder eine Einfachbindung repräsentiert, R⁵, R⁶, R⁷, R⁸ Wasserstoff oder eine niedere Alkylgruppe repräsentieren sowie m, n, p, q Zahlen von 1 bis 4 sind und R⁴ ein Wasserstoffatom oder eine niedere Alkylgruppe repräsentiert,
c) 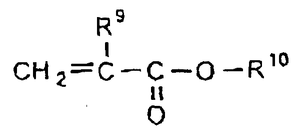
wobei R⁹ ein Wasserstoffatom oder eine niedere Alkylgruppe repräsentiert und R¹⁰ eine niedere Alkylgruppe repräsentiert und die genannten niederen Alkylgruppen oder Alkylengruppen 1 bis 6 Kohlenstoffatome aufweisen.

32. Copolymer nach Anspruch 31, **dadurch gekennzeichnet, daß** das Molekulargewicht zwischen 10 000 und 100 000 beträgt.

33. Verfahren zur Herstellung eines Copolymers gemäß Anspruch 31, **dadurch gekennzeichnet, daß** die Komponenten a), b), c), aufgeführt in Anspruch 31, mit Polymerisationsinititatorreagenzien wie Kaliumpersulfat, Ammoniumpersulfat, Benzophenon oder Methacryloyloxyhydroxylbenzophenon, polymerisiert werden.

## Revendications

1. Copolymère, **caractérisé en ce que** le copolymère est composé des motifs structuraux [I], [II] et [III] suivants, où il comprend 60 à 80 % en poids de [I], 10 à 30 % en poids de [II] et 5 à 20 % en poids de [III], sur la base du poids total de 100 %, 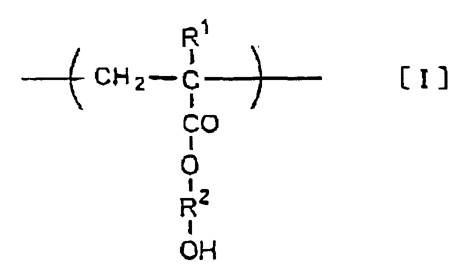 où R¹ représente un atome d'hydrogène ou un groupement alkyle inférieur, R² représente un groupement alkylène inférieur, et le groupement alkylène inférieur peut être substitué par un groupement hydroxyle, et il peut y avoir un atome d'oxygène dans la chaîne du groupement alkylène inférieur, 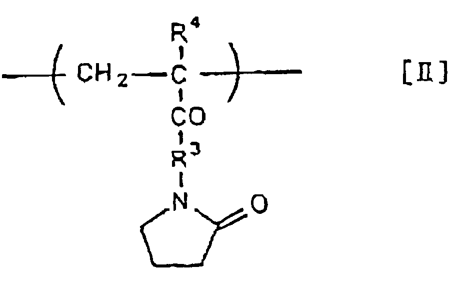 où R³ représente 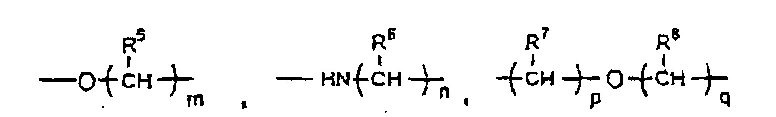 ou une simple liaison, où R⁵, R⁶, R⁷ et R⁸ représentent respectivement un atome d'hydrogène ou un groupement alkyle inférieur et m, n, p et q sont des nombres entiers de 1 à 4, et R⁴ représente un atome d'hydrogène ou un groupement alkyle inférieur, 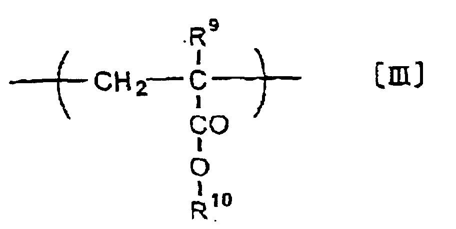 où R⁹ représente un atome d'hydrogène ou un groupement alkyle inférieur et R¹⁰ représente un groupement alkyle inférieur, lesdits groupements alkyle ou alkylène inférieurs comportant 1 à 6 atomes de carbone.

2. Copolymère selon la revendication 1, dans lequel ladite masse moléculaire va de dix mille à cent mille.

3. Copolymère selon la revendication 1, dans lequel ledit R¹ représente un atome d'hydrogène ou un groupement méthyle et R² représente -(CH₂)₂- ou -(CH₂)₃-.

4. Copolymère selon la revendication 1, dans lequel ledit R³ représente -OCH₂-, -OCH₂CH₂-, -NHCH₂- ou une simple liaison et R⁴ représente un atome d'hydrogène ou un groupement méthyle.

5. Copolymère selon la revendication 1, dans lequel ledit R⁹ représente un atome d'hydrogène ou un groupement méthyle et R¹⁰ représente un groupement méthyle ou isobutyle.

6. Copolymère selon la revendication 1, dans lequel ledit R¹ représente un atome d'hydrogène ou un groupement méthyle et R² représente -(CH₂)₂- ou -(CH₂)₃-, R³ représente - OCH₂-, -OCH₂CH₂-, -NHCH₂- ou une simple liaison, R⁴ représente un atome d'hydrogène ou un groupement méthyle, R⁹ représente un atome d'hydrogène ou un groupement méthyle et R¹⁰ représente un groupement méthyle ou isobutyle.

7. Copolymère selon la revendication 1, dans lequel R¹ représente un groupement méthyle, R² représente -(CH₂)₂-, R³ représente -OCH₂CH₂-, R⁴ représente un groupement méthyle, R⁹ représente un groupement méthyle et R¹⁰ représente un groupement méthyle.

8. Copolymère selon la revendication 1, dans lequel ladite teneur en [I] est de 65 à 75 % en poids, ladite teneur en [II] est de 15 à 25 % en poids et ladite teneur en [III] est de 5 à 15 % en poids, sur la base du poids total de 100 % en poids.

9. Copolymère selon la revendication 1, dans lequel ladite teneur en [I] est de 70 % en poids, ladite teneur en [II] est de 21 % en poids et ladite teneur en [III] est de 9 % en poids.

10. Copolymère selon la revendication 1, **caractérisé en ce que** le copolymère est composé des motifs structuraux [I], [II] et [III] suivants, où il comprend 60 à 80 % en poids de [I], 10 à 30 % en poids de [II] et 5 à 20 % en poids de [III], sur la base du poids total de 100 % en poids, où ledit R¹ représente un atome d'hydrogène ou un groupement méthyle, R² représente -(CH₂)₂- ou -(CH₂)₃-, R³ représente -OCH₂-, -OCH₂CH₂-, -NHCH₂- ou une simple liaison, R⁴ représente un atome d'hydrogène ou un groupement méthyle, R⁹ représente un atome d'hydrogène ou un groupement méthyle et R¹⁰ représente un groupement méthyle ou isobutyle.

11. Copolymère selon la revendication 1, dans lequel ladite teneur en [I] est de 70 % en poids, ladite teneur en [II] est de 21 % en poids et ladite teneur en [III] est de 9 % en poids, et R¹ représente un groupement méthyle, R² représente un groupement éthylène, R³ représente -OCH₂CH₂-, R⁴ représente un groupement méthyle, R⁹ représente un groupement méthyle et R¹⁰ représente un groupement méthyle.

12. Copolymère selon la revendication 1, dans lequel un absorbeur UV est contenu.

13. Copolymère selon la revendication 12, **caractérisé en ce que** la forme contenue d'absorbeur UV est une combinaison chimique avec le copolymère.

14. Copolymère selon la revendication 13, dans lequel ladite teneur en absorbeur UV est de 0,5 à 2,0 % en poids du copolymère.

15. Copolymère selon la revendication 13, dans lequel ladite teneur en absorbeur UV est de 0,8 à 1,5 % en poids du copolymère.

16. Copolymère selon la revendication 12, dans lequel ledit absorbeur est l'hydroxybenzophénone.

17. Copolymère selon la revendication 12, dans lequel ledit absorbeur UV est l'un des composés parmi la série des acides acryliques comprenant un groupement résiduel hydroxybenzophénone ou benzotriazole.

18. Copolymère selon la revendication 1 ou 12, dans lequel il est réticulé avec ledit agent de réticulation de composés de la série des acrylates ou de la série des acrylamides.

19. Copolymère selon la revendication 18, dans lequel ledit agent de réticulation est choisi parmi le bisméthacrylate d'éthylène glycol, le bisméthacrylate de diéthylène glycol ou le N,N'-méthylène-bisacrylamide et la teneur en agent de réticulation est de 0,01 à 2,0 % en poids du copolymère.

20. Copolymère selon la revendication 18, dans lequel ladite teneur en agent de réticulation est de 0,1 à 1,5 % en poids du copolymère.

21. Copolymère selon la revendication 18, dans lequel ladite teneur en agent de réticulation est de 0,5 à 1,0 % en poids du copolymère.

22. Copolymère selon l'une quelconque des revendications 1, 2 ou 18, dans lequel ledit polysaccharide est utilisé pour traiter la surface du copolymère.

23. Copolymère selon la revendication 22, dans lequel ledit traitement de surface est réalisé par la liaison covalente entre le copolymère et le polysaccharide.

24. Copolymère selon la revendication 22, dans lequel ladite liaison covalente est formée en utilisant la divinylsulfone.

25. Copolymère selon la revendication 22, dans lequel ledit polysaccharide est l'héparine, l'acide hyaluronique ou son sel.

26. Lentille oculaire qui est formée à partir du copolymère selon l'une quelconque des revendications 1, 12, 18 et 22.

27. Lentille oculaire pliable qui est formée à partir du copolymère selon l'une quelconque des revendications 1, 12, 18 et 22.

28. Lentille oculaire pliable qui est formée à partir du copolymère selon l'une quelconque des revendications 1, 12, 18 et 22 et qui présente les caractéristiques suivantes :
a) l'indice de réfraction est de 1,4 à 1,6,
b) la vitesse de rétablissement de l'état plié à l'état d'origine est de 3 à 6 secondes,
c) lorsqu'elle est gonflée par l'humidité, la résistance à la traction est supérieure à 10 livres par pouce carré (6,895 X 10⁴ Pa).

29. Lentille oculaire pliable qui est formée à partir du copolymère selon la revendication 1, dans laquelle ledit absorbeur UV est contenu, un agent de réticulation est utilisé pour réticuler le copolymère et un polysaccharide est utilisé pour traiter la surface du copolymère.

30. Lentille oculaire pliable selon la revendication 29, dans laquelle ledit absorbeur UV est choisi parmi tout type quelconque de composés de la série des benzophénones, de composés de la série des benzotriazoles ou de composés de la série des acides acryliques comprenant un groupement résiduel hydroxybenzophénone ou benzotriazole, ledit agent de réticulation est choisi parmi des composés de la série des acrylates ou des composés de la série des acrylamides et le polysaccharide est choisi parmi l'héparine, l'acide hyaluronique ou son sel.

31. Copolymère qui est copolymérisé à partir des composants a), b) et c) suivants :
a) 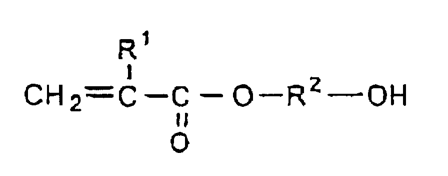 où R¹ représente un atome d'hydrogène ou un groupement alkyle inférieur, R² représente un groupement alkylène inférieur, qui peut être substitué par un groupement hydroxyle, et il peut y avoir un atome d'oxygène dans la chaîne du groupement alkylène,
b) 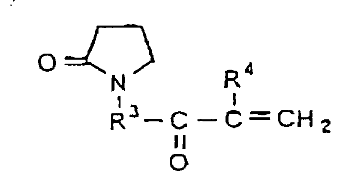 Où R³ représente 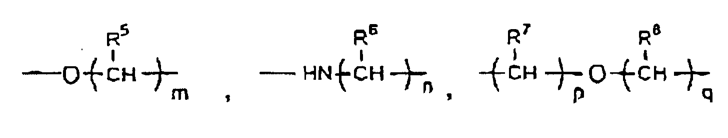 ou une simple liaison, où R⁵, R⁶, R⁷ et R⁸ représentent respectivement un atome d'hydrogène ou un groupement alkyle inférieur et m, n, p et q sont des nombres entiers de 1 à 4, et R⁴ représente un atome d'hydrogène ou un groupement alkyle inférieur,
c) 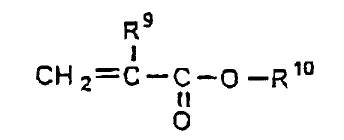 où R⁹ représente un atome d'hydrogène ou un groupement alkyle inférieur et R¹⁰ représente un groupement alkyle inférieur, lesdits groupements alkyle ou alkylène inférieurs comportant 1 à 6 atomes de carbone.

32. Copolymère selon la revendication 31, dans lequel la masse moléculaire va de dix mille à cent mille.

33. Procédé de préparation d'un copolymère selon la revendication 31,
**caractérisé en ce que** les composants a), b) et c) selon la revendication 31 sont polymérisés avec un agent d'amorçage de polymérisation tel que le persulfate de potassium, le persulfate d'ammonium, la benzophénone ou la méthacryloyloxyhydroxybenzophénone.
